(19) **Europäisches Patentamt / European Patent Office / Office européen des brevets**

(11) **EP 4 257 682 A1**

(12) **EUROPEAN PATENT APPLICATION**
published in accordance with Art. 153(4) EPC

(43) Date of publication:
**11.10.2023 Bulletin 2023/41**

(21) Application number: **21900665.7**

(22) Date of filing: **02.12.2021**

(51) International Patent Classification (IPC):
***C12N 9/80*** (2006.01)

(52) Cooperative Patent Classification (CPC):
**C12N 9/80**

(86) International application number:
**PCT/JP2021/044245**

(87) International publication number:
**WO 2022/118914 (09.06.2022 Gazette 2022/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.12.2020 JP 2020201413**

(71) Applicant: **Amano Enzyme Inc.**
**Nagoya-shi**
**Aichi 460-8630 (JP)**

(72) Inventors:
• **NORIFUNE, Saki**
**Konan-shi, Shiga 520-3203 (JP)**
• **TANAKA, Yusuke**
**Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

(54) **LIQUID ENZYME PREPARATION**

(57) The present invention provides a liquid enzyme preparation of a protein deamidase having excellent activity stability. This liquid enzyme preparation that contains a protein deamidase and at least 30 w/v% of sorbitol and that has a pH of 5.5 or more can enhance the stability of protein deamidase activity.

EP 4 257 682 A1

**Description**

TECHNICAL FIELD

The present invention relates to a liquid enzyme preparation. More specifically, the present invention relates to a liquid enzyme preparation of a protein deamidase having excellent activity stability.

BACKGROUND ART

As an enzyme that deamidates a γ-amide group and a β-amide group of a glutamine residue and an asparagine residue in a protein, as described in Patent Document 1 and the like, a protein glutaminase derived from Chryseobacterium gleum JCM2410 strain and a protein glutaminase derived from Chryseobacterium proteolyticum 9670 strain are known. Enzyme preparation products of such protein deamidases are currently only commercially available in a powder form.

As for the enzyme preparation, generally, a liquid form is more desirable than a powder form in consideration of dusting and handling. For this reason, a technique for formulating an enzyme preparation in a liquid form has been studied for various enzymes. For example, Patent Document 2 proposes a liquid enzyme preparation containing at least one milk protein crosslinking and/or modifying enzyme in polyol-water suspension containing 25% to 100% (w/w) polyol and having a pH value within a range from 4.4 to 5.1. Specifically, Patent Document 2 discloses that the stability of transglutaminase at a pH of 5.2 is low, and based on this finding, the stability of a transglutaminase preparation in a glycerol-water suspension with a pH of 4.4 to 4.8 and a transglutaminase preparation in a sorbitol-water suspension with a pH of 4.6 is improved, and in addition thereto, also discloses a tyrosinase preparation in a glycerol-water suspension with a pH of 4.6 and a protein glutaminase preparation in a glycerol-water suspension with a pH of 4.6.

PRIOR ART DOCUMENT

PATENT DOCUMENTS

Patent Document 1: WO 2010/029685 A
Patent Document 2: WO 2013/064736 A

SUMMARY OF THE INVENTION

PROBLEMS TO BE SOLVED BY THE INVENTION

In Patent Document 2, the stability of the liquid enzyme preparation of the transglutaminase preparation has been studied, while a liquid enzyme preparation of a protein deamidase has been only disclosed as a preparation formulation, and the stability of this enzyme has not been sufficiently studied.

Therefore, an object of the present invention is to provide a liquid enzyme preparation of a protein deamidase having excellent activity stability.

MEANS FOR SOLVING THE PROBLEM

The present inventors have conducted intensive studies, and as a result, have found that the activity stability of the protein deamidase is improved by a new formulation in which the pH is adjusted to 5.5 or more and 30 w/v% or more of sorbitol is blended. The present invention has been completed based on this finding. The present invention includes the following inventions.

Item 1. A liquid enzyme preparation containing a protein deamidase and 30 w/v% or more of sorbitol and having a pH of 5.5 or more.
Item 2. The liquid enzyme preparation described in item 1, in which a content of the sorbitol is 65 w/v% or less.
Item 3. The liquid enzyme preparation described in item 1 or 2, in which the pH is 8.2 or less.
Item 4. The liquid enzyme preparation described in any one of claims 1 to 3, further containing a sulfite, a thiosulfate, and/or a pyrosulfate.
Item 5. The liquid enzyme preparation described in any one of items 1 to 4, in which protein deamidase activity is 0.1 to 10,000 U/ml.

Item 6. The liquid enzyme preparation described in any one of items 1 to 5, in which the protein deamidase is derived from Chryseobacterium proteolyticum.

Item 7. An enzyme activity stabilizer for a liquid enzyme preparation of a protein deamidase, containing sorbitol as an active ingredient, the enzyme activity stabilizer being used at a concentration of 30 w/v% or more in the liquid enzyme preparation of the protein deamidase with a pH of 5.5 or more.

Item 8. The enzyme activity stabilizer for a liquid enzyme preparation of a protein deamidase described in item 7, further containing a sulfite, a thiosulfate, and/or a pyro sulfate.

Item 9. A precipitation inhibitor for a liquid enzyme preparation of a protein deamidase, containing sorbitol as an active ingredient, the precipitation inhibitor being used at a concentration of 30 w/v% or more in the liquid enzyme preparation of the protein deamidase with a pH of 5.5 or more.

## ADVANTAGES OF THE INVENTION

According to the present invention, there is provided a liquid enzyme preparation of a protein deamidase having excellent activity stability.

## BRIEF DESCRIPTION OF THE DRAWING

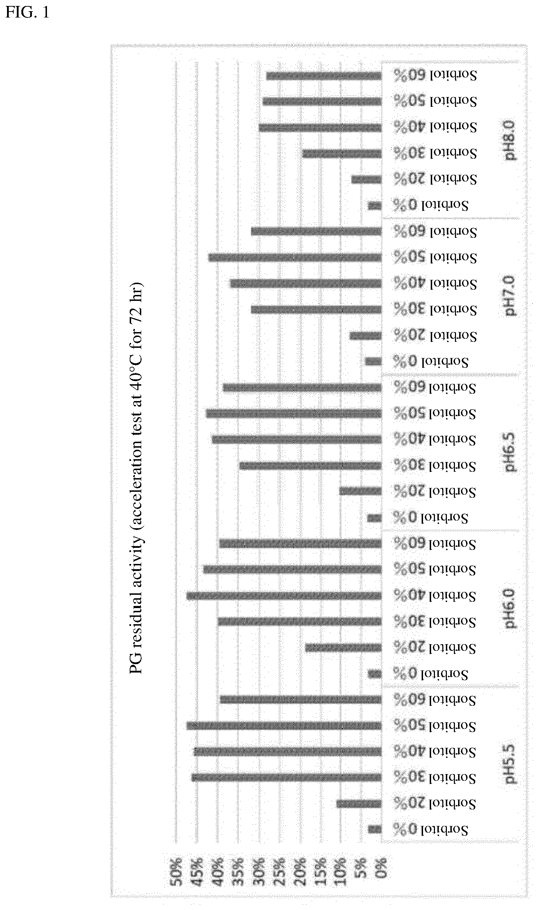

Fig. 1 shows test results of the activity stability of a liquid enzyme preparation of a protein deamidase (protein glutaminase: PG) obtained in Test Example 2.

## EMBODIMENTS OF THE INVENTION

[1. Liquid Enzyme Preparation]

A liquid enzyme preparation of the present invention contains a protein deamidase and 30 w/v% or more of sorbitol and has a pH of 5.5 or more. Hereinafter, the liquid enzyme preparation of the present invention will be described in detail.

[1-1. Protein Deamidase]

The type, origin, and the like of the protein deamidase are not particularly limited as long as the protein deamidase is an enzyme that exhibits an action of decomposing an amide group-containing side chain of a protein without cleaving peptide bonds and crosslinking the protein. As long as the above action is main activity, the protein deamidase may further has an action of decomposing an amide group-containing side chain of a protein with cleaving peptide bonds and crosslinking the protein. Examples of the protein deamidase include an enzyme that deamidates a glutamine residue in a protein and converts it into glutamic acid (for example, protein glutaminase), and an enzyme that deamidates an asparagine residue in a protein and converts it into aspartic acid (for example, protein asparaginase). More specific examples of the protein deamidase include commercially available products of a protein deamidase derived from the genus Chryseobacterium, Flavobacterium, Empedobacter, Sphingobacterium, Aureobacterium, or Myroides, which is disclosed in JP 2000-50887 A, JP 2001-218590 A, and WO 2006/075772 A1, a protein deamidase derived from the genus Luteimicrobium, Agromyces, Microbacterium, or Leifsonia, which is disclosed in WO 2015/133590 A1, and a protein glutaminase derived from the genus Chryseobacterium. These protein deamidases may be used singly or in combination of a plurality of kinds thereof.

Among these protein deamidases, from the viewpoint of obtaining a liquid enzyme preparation having further excellent activity stability, a protein deamidase derived from the genus Chryseobacterium is preferable, a protein glutaminase derived from the genus Chryseobacterium is more preferable, and a protein glutaminase derived from Chryseobacterium proteolyticum species is further preferable.

The protein deamidase can be prepared from a culture solution of a microorganism from which the protein deamidase is derived. Specific examples of the preparation method include a method of recovering a protein deamidase from a culture solution or a bacterial cell of the above-mentioned microorganism. For example, in the case of using a microorganism that secretes a protein deamidase, an enzyme can be separated and/or purified after recovering bacterial cells from the culture solution in advance by filtration, a centrifugal treatment, or the like, as necessary. In the case of using a microorganism that does not secretes a protein deamidase, an enzyme can be separated and/or purified after recovering bacterial cells from the culture solution in advance as necessary and then disrupting the bacterial cells by a pressurization treatment, an ultrasonic treatment, or the like to expose an enzyme. As an enzyme separation and/or purification method, a known protein separation and/or purification method can be used without particular limitation, and examples thereof include a centrifugal separation method, a UF concentration method, a salting-out method, and various

3

chromatography methods using an ion-exchange resin or the like.

The content of the protein deamidase in the liquid enzyme preparation of the present invention is not particularly limited, and is, for example, 0.1 to 10,000 U/mL, 1 to 5,000 U/mL, 10 to 2,500 U/ml, or 50 to 1,000 U/ml, preferably 100 to 800 U/ml, more preferably 150 to 700 U/ml, further preferably 250 to 600 U/ml or 250 to 500 U/ml, and even more preferably 300 to 400 U/ml.

For the activity of the protein deamidase, benzyloxycarbonyl-L-glutaminylglycine (Z-Gln-Gly) is used as a substrate, and the amount of enzyme that liberates 1 μmol of ammonia per minute is defined as 1 unit (1 U).

[1-2. Sorbitol]

The liquid enzyme preparation of the present invention contains sorbitol. The sorbitol is blended for the purpose of stabilizing the activity of the protein deamidase.

The content of the sorbitol in the liquid enzyme preparation of the present invention is 30 w/v% or more from the viewpoint of obtaining desired activity stability. In the liquid enzyme preparation of the present invention, the used amount of the sorbitol per 1 U of the protein deamidase is determined depending on the respective contents of the protein deamidase and the sorbitol, and is preferably 0.8 mg or more from the viewpoint of obtaining desired activity stability.

From the viewpoint of exhibiting more excellent activity stability, the content of the sorbitol in the liquid enzyme preparation of the present invention may be preferably 35 w/v% or more or 38 w/v% or more. Similarly, from the viewpoint of exhibiting more excellent activity stability, the used amount of the sorbitol per 1 U of the protein deamidase in the liquid enzyme preparation of the present invention may be preferably 0.97 mg or more or 1 mg or more. Examples corresponding to these cases include a case where the pH (pH at 25°C; the same applies hereinafter) of the liquid enzyme preparation of the present invention is 5.8 to 8.2 and preferably 6.0 to 8.0.

From the viewpoint of exhibiting further excellent activity stability, the content of the sorbitol in the liquid enzyme preparation of the present invention may be preferably 45 w/v% or more or 48 w/v% or more. Similarly, from the viewpoint of exhibiting further excellent activity stability, the used amount of the sorbitol per 1 U of the protein deamidase in the liquid enzyme preparation of the present invention may be preferably 1.2 mg or more or 1.3 mg or more. Examples corresponding to these cases include a case where the pH of the liquid enzyme preparation of the present invention is 6.5 to 7.5, preferably 6.8 to 7.2, and further preferably 6.9 to 7.1.

The upper limit of the content range of the sorbitol in the liquid enzyme preparation of the present invention is not particularly limited, and is, for example, 65 w/v% or less, preferably 60 w/v% or less, more preferably 57 w/v% or less, further preferably 55 w/v% or less, and even more preferably 52 w/v% or less, from the viewpoint of exhibiting more excellent activity stability and/or the viewpoint of ease of preparation. Similarly, the upper limit of the used amount range of the sorbitol per 1 U of the protein deamidase in the liquid enzyme preparation of the present invention is not particularly limited, and is, for example, 1.8 mg or less, preferably 1.67 mg or less, more preferably 1.59 mg or less, further preferably 1.53 mg or less, and even more preferably 1.4 mg or less, from the viewpoint of exhibiting more excellent activity stability and/or the viewpoint of ease of preparation.

From the viewpoint of exhibiting further excellent activity stability, the upper limit of the content range of the sorbitol in the liquid enzyme preparation of the present invention may be preferably 50 w/v% or less, more preferably 47 w/v% or less, further preferably 45 w/v% or less, and even more preferably 42 w/v% or less. Similarly, from the viewpoint of exhibiting further excellent activity stability, the upper limit of the used amount range of the sorbitol per 1 U of the protein deamidase in the liquid enzyme preparation of the present invention may be preferably 1.4 mg or less, more preferably 1.3 mg or less, further preferably 1.25 mg or less, and even more preferably 1.16 mg or less. Examples corresponding to these cases include a case where the pH of the liquid enzyme preparation of the present invention is 5.5 to 8.0, preferably 5.5 to 7.2, more preferably 5.8 or more and less than 6.5, further preferably 5.9 to 6.3, and even more preferably 6.0 to 6.2.

In a specific embodiment of the present invention, 30 w/v% or more of sorbitol in the liquid enzyme preparation may be blended for the purpose of suppressing precipitation in addition to stabilizing the activity. In this embodiment, from the viewpoint of enhancing the precipitation suppressing effect, the content of the sorbitol in the liquid enzyme preparation of the present invention is preferably 38 w/v% or more or 45 w/v% or more, more preferably 48 w/v% or more or 55 w/v% or more, and further preferably 58 w/v% or more. Similarly, in a specific embodiment of the present invention, 0.8 mg or more of sorbitol per 1 U of the protein deamidase in the liquid enzyme preparation may be blended for the purpose of suppressing precipitation in addition to stabilizing the activity. In this embodiment, from the viewpoint of enhancing the precipitation suppressing effect, the used amount of the sorbitol per 1 U of the protein deamidase in the liquid enzyme preparation of the present invention is preferably 1 mg or more or 1.25 mg or more, more preferably 1.3 mg or more or 1.5 mg or more, and further preferably 1.6 mg or more. Examples corresponding to these embodiments include a case where the pH of the liquid enzyme preparation of the present invention is 5.5 or more and less than 6.75 and more preferably 5.5 to 6.5.

In another specific embodiment of the present invention, 38 w/v% or more or 45 w/v% or more of sorbitol in the liquid enzyme preparation of the present invention may be blended for the purpose of suppressing precipitation in addition to stabilizing the activity. In this embodiment, from the viewpoint of enhancing the precipitation suppressing effect, the content of the sorbitol in the liquid enzyme preparation of the present invention is preferably 48 w/v% or more or 55 w/v% or more and more preferably 58 w/v% or more. Similarly, in another specific embodiment of the present invention, 1 mg or more or 1.25 mg or more of sorbitol per 1 U of the protein deamidase in the liquid enzyme preparation of the present invention may be blended for the purpose of suppressing precipitation in addition to stabilizing the activity. In this embodiment, from the viewpoint of enhancing the precipitation suppressing effect, the used amount of the sorbitol per 1 U of the protein deamidase in the liquid enzyme preparation of the present invention is preferably 1.3 mg or more or 1.5 mg or more and more preferably 1.6 mg or more. Examples corresponding to these embodiments include a case where the pH of the liquid enzyme preparation of the present invention is 6.75 or more and less than 7.5 and more preferably 6.8 to 7.2.

In still another specific embodiment of the present invention, 48 w/v% or more or 55 w/v% or more of sorbitol in the liquid enzyme preparation of the present invention may be blended for the purpose of suppressing precipitation in addition to stabilizing the activity. In this embodiment, from the viewpoint of enhancing the precipitation suppressing effect, the content of the sorbitol in the liquid enzyme preparation of the present invention is preferably 58 w/v% or more. Similarly, in another specific embodiment of the present invention, 1.3 mg or more or 1.5 mg or more of sorbitol per 1 U of the protein deamidase in the liquid enzyme preparation of the present invention may be blended for the purpose of suppressing precipitation in addition to stabilizing the activity. In this embodiment, from the viewpoint of enhancing the precipitation suppressing effect, the used amount of the sorbitol per 1 U of the protein deamidase in the liquid enzyme preparation of the present invention is preferably 1.6 mg or more. Examples corresponding to these embodiments include a case where the pH of the liquid enzyme preparation of the present invention is 7.5 or more.

[1-3. Sulfite, Thiosulfate, and Pyrosulfate]

The liquid enzyme preparation of the present invention preferably further contains a sulfite, a thiosulfate, and/or a pyrosulfate as a predetermined salt from the viewpoint of exhibiting further excellent activity stability. Specific examples of these predetermined salts include metal salts of alkali metal salts (such as a potassium salt and a sodium salt) and alkaline earth metal salts (such as a calcium salt). These predetermined salts may be used singly or in combination of a plurality of kinds thereof.

Among these, from the viewpoint of exhibiting further excellent activity stability, a sulfite and a pyrosulfate are more preferable, a sulfite is even more preferable, or an alkali metal salt is more preferable and a sodium salt is further preferable. Among these, from the viewpoint of exhibiting particularly excellent activity stability, an alkali metal sulfite is particularly preferable and sodium sulfite is most preferable.

When the liquid enzyme preparation of the present invention contains the predetermined salt, the blending amount of the predetermined salt is not particularly limited, and may be appropriately set according to the effect of improving the activity stabilization to be imparted, and the blending amount thereof is, for example, 0.01 to 2 w/v%, preferably 0.03 to 1 w/v%, preferably 0.04 to 0.8 w/v%, more preferably 0.06 to 0.6 w/v% or 0.08 to 0.6 w/v%, even more preferably 0.15 to 0.55 w/v%, 0.2 to 0.55 w/v%, 0.3 to 0.55 w/v%, or 0.4 to 0.55 w/v%. Alternatively, the blending amount of the predetermined salt per 1 U of the protein deamidase is, for example, 0.00025 to 0.05 mg, preferably 0.0008 to 0.026 mg, preferably 0.001 to 0.023 mg, more preferably 0.0015 to 0.017 mg or 0.002 to 0.017 mg, and even more preferably 0.004 to 0.015 mg, 0.005 to 0.015 mg, 0.008 to 0.015 mg, or 0.01 to 0.015 mg.

[1-4. Other Components]

The liquid enzyme preparation of the present invention may contain other components to the extent that the effect of the present invention is not affected, in addition to the protein deamidase and the sorbitol described above. Examples of the other components include enzymes other than the protein deamidase and additives.

Examples of the other enzymes include amylase ($\alpha$-amylase, $\beta$-amylase, or glucoamylase), glucosidase ($\alpha$-glucosidase or $\beta$-glucosidase), galactosidase ($\alpha$-galactosidase or $\beta$-galactosidase), protease (acidic protease, neutral protease, or alkaline protease), peptidase (leucine peptidase or aminopeptidase), lipase, esterase, cellulase, phosphatase (acid phosphatase or alkaline phosphatase), nuclease, deaminase, oxidase, dehydrogenase, glutaminase, pectinase, catalase, dextranase, transglutaminase, and pullulanase. These other enzymes may be contained singly or in combination of a plurality of kinds thereof.

Examples of the additive include excipients, buffers, suspending agents, stabilizers, preservatives, antiseptics, physiological saline, polyhydric alcohols (referring to polyhydric alcohols other than the sorbitol; the same applies hereinafter), pH adjusters. These additives may be contained singly or in combination of a plurality of kinds thereof.

Among these additives, from the viewpoint of further improving the activity stability of the liquid enzyme preparation of the present invention, the liquid enzyme preparation of the present invention preferably does not substantially contain a polyhydric alcohol. The polyhydric alcohol includes a non-sugar polyhydric alcohol such as glycerin or propylene glycol, and a sugar alcohol other than sorbitol. The expression "does not substantially contain a polyhydric alcohol" means that the liquid enzyme preparation of the present invention is allowed to contain a polyhydric alcohol to such an extent that the activity stability of the liquid enzyme preparation is not impaired, and specifically, the content of the polyhydric alcohol is 1 w/v% or less, preferably 0.1 w/v% or less, more preferably 0.01 w/v% or less, and most preferably 0 w/v%.

[1-5. Preparation Form etc.]

The liquid enzyme preparation of the present invention is a liquid preparation. The liquid enzyme preparation of the present invention can be prepared by adding a predetermined amount of each component to be blended to water to be dissolved, or removing unnecessary components such as contaminant polymers from a culture solution of a protein deamidase producing strain by separation and/or purification, adding a sorbitol aqueous solution, then appropriately adding a pH adjuster to adjust the pH to a predetermined pH, and/or blending a sulfite, a thiosulfate, and/or a pyrosulfate as necessary, and further sterilizing the mixture as necessary.

The pH (25°C) of the liquid enzyme preparation of the present invention is 5.5 or more from the viewpoint of obtaining desired activity stability. The upper limit of the pH range of the liquid enzyme preparation of the present invention is not particularly limited, and is 8.2 or less, preferably 8.0 or less, more preferably 7.6 or less, further preferably 7.2 or less, and even more preferably 7.0 or less, 6.8 or less, 6.5 or less, 6.2 or less, 6.0 or less, or 5.7 or less, from the viewpoint of exhibiting further excellent activity stability.

[1-6. Use Application]

As the use application of the liquid enzyme preparation of the present invention, the liquid enzyme preparation can be used for any use application utilizing protein deamidase activity. For example, the liquid enzyme preparation of the present invention can be used for the purpose of modifying a protein. A specific embodiment of the modification of the protein is not particularly limited, and any modification may be used as long as it utilizes a change in properties of the protein caused by generation of a carboxyl group by deamidation of a $\gamma$-amide group and a $\beta$-amide group of a glutamine residue and an asparagine residue of the protein. Specifically, examples of the modification of the protein include an increase in solubility of the protein, an increase in water dispersibility, an improvement in emulsifying power, and emulsion stability.

Therefore, the protein deamidase liquid enzyme preparation of the present invention can be widely used in the food field. Specific examples of the use application include use application for improving solubility, dispersibility, emulsifiability, and the like of an animal protein and/or a plant protein in a weakly acidic condition environment in a pH range of normal foods (for example, use application for producing of acidic beverages such as coffee/whiteners and juices, dressings, mayonnaise, and creams); increase in solubility and dispersibility of a poorly soluble plant protein (for example, use application for producing a fried food powder or the like using wheat gluten); use application for modifying dough in bread making and baking (for example, use application for producing crackers, biscuits, cookies, pizza, or pie crust); use application for removing or reducing allergen in allergic protein in food (for example, use application for producing food for wheat allergic patients); use application for reducing mineral sensitivity of a protein, increasing a soluble mineral content in a liquid containing a protein and mineral, and enhancing absorbability of the mineral into a human body (for example, use application for producing highly mineral (for example, calcium)-containing beverages, mineral (for example, calcium) absorption enhancers); use application for reducing bitterness, use application for improving the proteolysis rate of a protease, and/or use application for enhancing the glutamic acid content (for example, amino acid-based seasoning (hydrolyzate of animal protein (HAP), hydrolyzate of plant protein (HVP)), and use application for producing fermented soybean paste or soy sauce).

[2. Enzyme Activity Stabilizer for Liquid Enzyme Preparation of Protein Deamidase and Precipitation Inhibitor for Liquid Enzyme Preparation of Protein Deamidase]

As described above, in the liquid enzyme preparation containing a protein deamidase and having a pH of 5.5 or more, the sorbitol blended at a concentration of 30 w/v% or more improves the activity stability of the liquid enzyme preparation, that is, the stability of the protein deamidase activity. Therefore, the present invention also provides an enzyme activity stabilizer for a liquid enzyme preparation of a protein deamidase, containing sorbitol as an active ingredient, the enzyme activity stabilizer being used at a concentration of 30 w/v% or more in the liquid enzyme preparation of the protein deamidase with a pH of 5.5 or more. The enzyme activity stabilizer for the liquid enzyme preparation of the protein deamidase of the present invention preferably further contains a sulfite, a thiosulfate, and/or a pyrosulfate as a predetermined salt, in addition to the sorbitol, from the viewpoint of exhibiting further excellent activity stability.

As described above, in the liquid enzyme preparation containing a protein deamidase and having a pH of 5.5 or more, the sorbitol blended at a concentration of 30 w/v% or more can suppress precipitation of the liquid enzyme preparation. Therefore, the present invention also provides a precipitation inhibitor for a liquid enzyme preparation of a protein deamidase, containing sorbitol as an active ingredient, the precipitation inhibitor being used at a concentration of 30 w/v% or more in the liquid enzyme preparation of the protein deamidase with a pH of 5.5 or more.

More preferably, the precipitation inhibitor of the present invention is used at a concentration of 30 w/v% or more in the liquid enzyme preparation of the protein deamidase with a pH of 5.5 or more and less than 6.75, at a concentration of 38 w/v% or more in the liquid enzyme preparation of the protein deamidase with a pH of 6.75 or more and less than 7.5, or at a concentration of 48 w/v% or more in the liquid enzyme preparation of the protein deamidase with a pH of 7.5 or more.

In the enzyme activity stabilizer for the liquid enzyme preparation of the protein deamidase and the precipitation inhibitor for the liquid enzyme preparation of the protein deamidase of the present invention, the kind, used amount, and the like of each component are as described in the section "1. Liquid Enzyme Preparation" above.

EXAMPLES

Hereinafter, the present invention will be more specifically described by means of Examples; however, the present invention is not limited to these Examples.

[Test Example 1]

Unnecessary components such as contaminant polymers were removed from the culture solution of Chryseobacterium proteolyticum strain that was a protein glutaminase producing strain, a sorbitol aqueous solution and/or a glycerin aqueous solution was added, and then the pH was adjusted to 5.0 or 6.0 using 1 N hydrochloric acid or a 1 N sodium hydroxide aqueous solution to prepare a liquid enzyme preparation in which the concentrations of protein glutaminase (PG) as the protein deamidase, sorbitol, and glycerin were as shown in Table 1.

The obtained liquid enzyme preparation was left to stand at 60°C for 72 hours. Protein deamidase activity values before and after standing were measured by the following method.

To 1 mL of a 0.2 M phosphate buffer (pH 6.5) containing 30 mM Z-Gln-Gly, 0.1 mL of a sample solution containing a protein deamidase was added, the mixture was left to stand at 37°C for 10 minutes, and then 1 mL of a 0.4 M TCA solution was added to stop the reaction. As a blank, to 1 mL of a 0.2 M phosphate buffer (pH 6.5) containing 30 mM Z-Gln-Gly, 1 mL of a 0.4 M TCA solution was added, 0.1 mL of a sample solution containing a protein deamidase was further added, and the mixture was left to stand at 37°C for 10 minutes.

The amount of ammonia generated in the reaction solution was measured for the solution obtained above using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).

The activity of the protein deamidase was calculated from the following formula with the amount of enzyme that produces 1 μmol of ammonia per minute being defined as 1 unit (1 U). In the formula, the reaction solution amount is 2.1, the enzyme solution amount is 0.1, and Df is a dilution rate of the enzyme solution. 17.03 is a molecular weight of ammonia.

[Mathematical Formula 1]

Protein deamidase activity (U/mL)

$$= \text{Ammonia concentration in reaction solution (mg/l)} \times (1/17.03) \times (\text{Reaction}$$

$$\text{solution amount/Enzyme solution amount}) \times (1/10) \times Df$$

When the protein deamidase activity value before standing was regarded as 100%, a relative value (%) of the protein deamidase activity value after standing was calculated as residual activity. The results are shown in Table 1.

[Table 1]

| | PG (U/ml) | Sorbitol (w/v%) | Glycerin (w/v%) | pH (25 °C) | Residual activity at 60°C for 72 hr |
|---|---|---|---|---|---|
| Comparative Example 1 | 360 | - | - | 6.0 | 2% |
| Comparative Example 2 | 360 | - | 10 | 6.0 | 4% |
| Comparative Example 3 | 360 | - | 20 | 6.0 | 8% |
| Comparative Example 4 | 360 | - | 30 | 6.0 | 14% |
| Comparative Example 5 | 360 | - | 40 | 6.0 | 14% |
| Comparative Example 6 | 360 | - | 50 | 6.0 | 11% |
| Comparative Example 7 | 360 | 20 | - | 6.0 | 14% |
| Comparative Example 8 | 360 | 30 | - | 5.0 | 14% |
| Example 1 | 360 | 30 | - | 6.0 | 45% |

As shown in Table 1, in all of a case where glycerin was blended in the liquid enzyme preparation of PG (Comparative Examples 2 to 6), a case where 20 w/v% of sorbitol was blended (Comparative Example 7), and a case where 30 w/v% of sorbitol was blended and the pH was adjusted to 5.0 (Comparative Example 8),the activity stability was not much improved as compared with Comparative Example 1, but in a case where sorbitol was blended at 30 w/v% and the pH was adjusted to 6.0 (Example 1), a remarkable improvement in the activity stability was recognized.

[Test Example 2]

A liquid enzyme preparation containing protein glutaminase (PG) as a protein deamidase and sorbitol (not containing glycerin) was prepared in the same manner as in Test Example 1, except that the pH was adjusted to 5.5, 6.0, 6.5, 7.0, or 8.0, and the blending amount of sorbitol was set to 0 w/v%, 20 w/v%, 30 w/v%, 40 w/v%, 50 w/v%, or 60 w/v%. The PG content in the liquid enzyme preparation was 360 U/ml. The residual activity of the obtained liquid enzyme preparation after being left to stand at 60°C for 72 hours was measured in the same manner as in Test Example 1. Results are shown in Fig. 1.

As shown in Fig. 1, in the liquid enzyme preparation containing a protein deamidase and 30 w/v% or more of sorbitol and having a pH of 5.5 or more, significant improvement in activity stability was recognized

[Test Example 3]

The liquid enzyme preparation obtained in Test Example 2 was dispensed into microtubes (volume: 2.0 ml) by 1 ml, the appearance after the liquid enzyme preparation being left to stand at 60°C for 72 hours was observed, and the presence or absence and degree of precipitation were evaluated based on the following criteria. A representative view of the appearance corresponding to each criterion is shown in Table 2. The results are shown in Table 3.

-: No precipitation was observed, and the liquid enzyme preparation was clear.
+: Slight precipitation was observed.
++: A small amount of precipitation was observed.
+++: A large amount of precipitation was observed.

[Table 2]

| - | + | ++ | +++ |
|---|---|---|---|
| | | | |

[Table 3]

| | | pH | | | | |
|---|---|---|---|---|---|---|
| | | pH5.5 | pH6.0 | pH6.5 | pH7.0 | pH8.0 |
| Sorbitol content (w/v) | 0% | +++ | +++ | +++ | +++ | +++ |
| | 20% | +++ | +++ | +++ | +++ | +++ |
| | 30% | ++ | ++ | ++ | +++ | +++ |
| | 40% | + | + | ++ | ++ | +++ |
| | 50% | − | − | + | + | ++ |
| | 60% | − | − | − | − | − |

As shown in Table 3, when sorbitol was not contained, a large amount of precipitation was generated, but when 30 w/v% or more of sorbitol was added, precipitation was suppressed. In particular, when the pH of the liquid enzyme preparation was 5.5, 6.0, and 6.5, an excellent precipitation inhibitory effect was observed by adding 30 w/v% or more of sorbitol, when the pH of the liquid enzyme preparation was 7.0, an excellent precipitation inhibitory effect was observed by adding 40 w/v% or more of sorbitol, and when the pH of the liquid enzyme preparation was 8.0, an excellent precipitation inhibitory effect was observed by adding 50 w/v% or more of sorbitol.

[Test Example 4]

The residual activity was measured in the same manner as in Test Example 1, except that liquid enzyme preparations were prepared by further blending sodium sulfite, sodium thiosulfate, sodium pyrosulfate, magnesium sulfate, disodium dihydrogen ethylenediaminetetraacetate, or potassium chloride with the liquid enzyme preparation of Example 1 prepared in Test Example 1 and the liquid enzyme preparation of Example 1 in an amount shown in Table 4, and the conditions under which these liquid enzyme preparations were left to stand were set to 40°C and 2 weeks. The results are shown in Table 4.

[Table 4]

| | Example | | | | | | | | | |
|---|---|---|---|---|---|---|---|---|---|---|
| | 1 | 2 | 3 | 4 | 5 | 6 | 7 | 8 | 9 | 10 |
| PG (U/mL) | 360 | 360 | 360 | 360 | 360 | 360 | 360 | 360 | 360 | 360 |

(continued)

| | Example | | | | | | | | | |
|---|---|---|---|---|---|---|---|---|---|---|
| | 1 | 2 | 3 | 4 | 5 | 6 | 7 | 8 | 9 | 10 |
| Sorbitol (w/v%) | 30 | 30 | 30 | 30 | 30 | 30 | 30 | 30 | 30 | 30 |
| Sodium sulfite (w/v%) | - | 0.01 | 0.05 | 0.1 | 0.5 | - | - | - | - | - |
| Sodium thiosulfate (w/v%) | - | - | - | - | - | 0.9 | - | - | - | - |
| Sodium pyrosulfate (w/v%) | - | - | - | - | - | - | 0.8 | - | - | - |
| Magnesium sulfate (w/v%) | - | - | - | - | - | - | - | 0.5 | - | - |
| EDTA-2Na (w/v%) | - | - | - | - | - | - | - | - | 0.5 | - |
| KCl (w/v%) | - | - | - | - | - | - | - | - | - | 0.5 |
| pH (25°C) | 6.0 | 6.0 | 6.0 | 6.0 | 6.0 | 6.0 | 6.0 | 6.0 | 6.0 | 6.0 |
| Residual activity at 40°C for 2 weeks | 70% | 77% | 91% | 96% | 100% | 85% | 92% | 69% | 69% | 58% |

As is apparent from Table 4, even when various salts were further blended in the liquid enzyme preparation with a pH of 6.0 containing protein glutaminase and 30 w/v% of sorbitol, the enzyme activity stabilizing effect was effectively obtained. In particular, when sodium sulfite, sodium thiosulfate, or sodium pyrosulfate was further blended, a higher enzyme activity stabilizing effect was obtained, and this tendency was remarkable when sodium sulfite and sodium pyrosulfate were blended, particularly when sodium sulfite was blended.

**Claims**

1. A liquid enzyme preparation comprising a protein deamidase and 30 w/v% or more of sorbitol and having a pH of 5.5 or more.

2. The liquid enzyme preparation according to claim 1, wherein a content of the sorbitol is 65 w/v% or less.

3. The liquid enzyme preparation according to claim 1 or 2, wherein the pH is 8.2 or less.

4. The liquid enzyme preparation according to any one of claims 1 to 3, further comprising a sulfite, a thiosulfate, and/or a pyrosulfate.

5. The liquid enzyme preparation according to any one of claims 1 to 4, wherein protein deamidase activity is 0.1 to 10,000 U/ml.

6. The liquid enzyme preparation according to any one of claims 1 to 5, wherein the protein deamidase is derived from Chryseobacterium proteolyticum.

7. An enzyme activity stabilizer for a liquid enzyme preparation of a protein deamidase, comprising sorbitol as an active ingredient, the enzyme activity stabilizer being used at a concentration of 30 w/v% or more in the liquid enzyme preparation of the protein deamidase with a pH of 5.5 or more.

8. The enzyme activity stabilizer for a liquid enzyme preparation of a protein deamidase according to claim 7, further comprising a sulfite, a thiosulfate, and/or a pyro sulfate.

9. A precipitation inhibitor for a liquid enzyme preparation of a protein deamidase, comprising sorbitol as an active ingredient, the precipitation inhibitor being used at a concentration of 30 w/v% or more in the liquid enzyme preparation of the protein deamidase with a pH of 5.5 or more.

FIG. 1

PG residual activity (acceleration test at 40°C for 72 hr)

## INTERNATIONAL SEARCH REPORT

| International application No. |
|---|
| **PCT/JP2021/044245** |

| A. | CLASSIFICATION OF SUBJECT MATTER |
|---|---|

*C12N 9/80*(2006.01)i
FI: C12N9/80 Z

According to International Patent Classification (IPC) or to both national classification and IPC

| B. | FIELDS SEARCHED |
|---|---|

Minimum documentation searched (classification system followed by classification symbols)

C12N9/80

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2019/049927 A1 (AMANO ENZYME INC.) 14 March 2019 (2019-03-14) claims, examples | 1-9 |
| A | JP 2014-532421 A (VALIO LTD.) 08 December 2014 (2014-12-08) paragraphs [0007]-[0010] | 1-9 |

☐ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 February 2022** | **15 February 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2021/044245**

| Patent document cited in search report | | | Publication date (day/month/year) | Patent family member(s) | | | Publication date (day/month/year) |
|---|---|---|---|---|---|---|---|
| WO | 2019/049927 | A1 | 14 March 2019 | US 2020/0283752 A1 claims, examples | | | |
| JP | 2014-532421 | A | 08 December 2014 | WO 2013/064736 A1 paragraphs [0023]-[0028] | | | |

Form PCT/ISA/210 (patent family annex) (January 2015)

**REFERENCES CITED IN THE DESCRIPTION**

**Patent documents cited in the description**

- WO 2010029685 A
- WO 2013064736 A
- JP 2000050887 A
- JP 2001218590 A
- WO 2006075772 A1
- WO 2015133590 A1